# NEUE EUROPÄISCHE PATENTSCHRIFT

(11) **EP 0 734 247 B2**
(45) Veröffentlichungstag und Bekanntmachung des Hinweises auf die Entscheidung über den Einspruch: **15.06.2005**
(45) Hinweis auf die Patenterteilung: 28.10.1998
(21) Anmeldenummer: 95904451.2
(22) Anmeldetag: 10.12.1994
(51) Int. Cl.: A61K 7/48

(54) **KOSMETISCHE UND/ODER PHARMAZEUTISCHE ZUBEREITUNGEN**
COSMETIC AND/OR PHARMACEUTICAL PREPARATIONS
PREPARATIONS COSMETIQUES ET/OU PHARMACEUTIQUES

(30) Priorität: 18.12.1993 DE 4343431
(43) Veröffentlichungstag der Anmeldung: 02.10.1996
(73) Patentinhaber: Cognis Deutschland GmbH & Co. KG, 40589 Düsseldorf (DE)
(72) Erfinder: ANSMANN, Achim, D-40699 Erkrath (DE); WADLE, Armin, D-40724 Hilden (DE); EILERS, Eberhard, D-89081 Ulm (DE); THOMAS, Heike, D-40764 Langenfeld (DE)
(86) Internationale Anmeldenummer: PCT/EP1994/004113
(87) Internationale Veröffentlichungsnummer: WO 1995/016430

(56) Entgegenhaltungen:
- EP-A- 0 203 418
- EP-A- 0 417 619
- WO-A-92/21318
- Firmenblatt "Lamepon S" der Henkel KG Firmenblatt "Lamepon S" der Henkel KG Cospha, Januar 1992 Cospha, Januar 1992
- Firmenschrift "Proteol VS 22" der Seppic, Firmenschrift "Proteol VS 22" der Seppic, Februar 1992 Februar 1992
- Karlheinz Schrader, Grundlagen und Karlheinz Schrader, Grundlagen und Rezepturen der Kosmetika, 2. Auflage, 1989, Rezepturen der Kosmetika, 2. Auflage, 1989, pp. 178-179, 687, 425 pp. 178-179, 687, 425

## Beschreibung

### Gebiet der Erfindung

Die Erfindung betrifft kosmetische und/oder pharmazeutische Zubereitungen mit einem Gehalt an hochacylierten Proteinhydrolysaten als Emulgatoren sowie die Verwendung dieser Stoffe als Emulgatoren für die genannten Mittel.

### Stand der Technik

Acylierungsprodukte von Proteinhydrolysaten, sogenannte Proteinfettsäurekondensate, stellen anionische Tenside dar, die wegen ihrer guten Reinigungsleistung und besonderen Hautverträglichkeit beispielsweise in wäßrigen kosmetischen Zubereitungen (Haarshampoos etc.) eingesetzt werden. Bezüglich der Eigenschaften dieser Tensidkiasse sei auf die Beiträge von G.Schuster und H.Modde in **Parf.Kosm. 45, 337 (1964)** und O.J. Muscio et al. in **J.Am.Oil.Chem.Soc. 59**, **217 (1982)** verwiesen.

EP-A-0 417 619 offenbart Proteinfettsäurekondensate, die durch Acylierung von Proteinhydrolysaten mit einer durchschnittlichen Molmasse von 3000 bis 7000 hergestellt werden.

Während Proteinfettsäurekondensate sich demnach in rein wäßrigen Systemen durch ausgezeichnete Eigenschaften auszeichnen, ist ihre Verwendung zusammen mit Ölkörpern in der Regel nicht möglich. Emulsionen, insbesondere O/W-Emulsionen mit einem Gehalt an acylierten Proteinhydrolysaten zeigen die Tendenz, mit zunehmender Lagerung einzudicken und sich zu entmischen.

Die Aufgabe der Erfindung hat somit darin bestanden, Emulsionen mit einem Gehalt an acylierten Proteinhydrolysaten zur Verfügung zu stellen, die auch bei längerer Lagerung stabil sind und eine konstante Viskosität aufweisen.

### Beschreibung der Erfindung

Gegenstand der Erfindung sind kosmetische und/oder pharmazeutische Zubereitungen, die sich dadurch auszeichnen, daß sie als Emulgatoren hochacylierte Proteinfettsäurekondensate enthalten, die einen Gesamtstickstoffgehalt - bezogen auf die Acylierungsprodukte - im Bereich von 2,0 bis 3,5 Gew.% aufweisen, wobei man Acylierungsprodukte von Sojaprotein-, Mandelprotein-, Weizenprotein-, und/oder Kartoffelprotein hydrolysaten einsetzt.

Überraschenderweise wurde gefunden, daß durch Anhebung des Acylierungsgrades, der über die Abnahme des Gesamtstickstoffgehaltes bestimmt werden kann, Proteinhydrolysate mit ausgezeichneten Emulgiereigenschaften erhalten werden. Die unter Verwendung dieser Stoffe hergestellten Emulsionen sind lagerstabil und weisen eine konstante Viskosität auf. Als besonders vorteilhaft hat sich dabei der Einsatz von Proteinfettsäurekondensaten auf Basis pflanzlicher Rohstoffe, insbesondere Sojaprotein erwiesen.

### Proteinbasis

Proteinfettsäurekondensate stellen wie erwähnt bekannte Stoffe dar. Auch zur Herstellung der neuen hochacylierten Produkte geht man üblicherweise pflanzlichen Proteinen, beispielsweise Mandelprotein, Weizenprotein, Kartoffelprotein und insbesondere Sojaprotein aus, die durch saure, alkalische und/oder enzymatische Hydrolyse gespalten werden und danach ein durchschnittliches Molekulargewicht im Bereich von 600 bis 4000, vorzugsweise 2000 bis 3500 aufweisen.

### Herstellung der hochacylierten Proteinfettsäurekondensate

Zur Herstellung von hochacylierten Proteinfettsäurekondensaten über den Weg der sauren Hydrolyse wird das Ausgangsprotein beispielsweise mit verdünnter Schwefelsäure versetzt und 8 bis 10 h bei 85 bis 95°C hydrolysiert. Nach Beendigung der Hydrolyse empfiehlt es sich, den Ansatz mit wäßriger Calciumhydroxidlösung zu versetzen, wodurch die Polypeptide in die Calciumsalze überführt werden und das Aufschlußmaterial als Calciumsulfat gefällt wird. Die Dispersion wird anschließend filtriert, wobei eine klare Lösung von Calciumpeptiden erhalten wird. Diese Lösung kann eingedampft und anschließend direkt mit Fettsäurechloriden acyliert werden. Sollen anstelle der Calciumsalze beispielsweise Natrium- oder Kaliumsalze hergestellt werden, empfiehlt es sich, die Calciumpeptidlösung vor der Schotten-Baumann-Reaktion mit Soda oder Pottasche zu behandeln, das ausgefällte Calciumcarbonat abzufiltrieren und anstelle der Calciumpeptide die Alkalipeptide weiterzuverarbeiten.

Zur Herstellung von hochacylierten Proteinfettsäurekondensaten über den Weg der alkalischen Hydrolyse wird das Ausgangsmaterial mit einer wäßrigen Calciumhydroxiddispersion versetzt, 6 bis 10 h bei 85 bis 95°C hydrolysiert und das aufgeschlossene Material vom Rückstand filtriert. Das Filtrat enthält die Calciumpeptide, die anschließend mit den Fettsäurechloriden acyliert werden können. Auch hier können die entsprechenden Alkalipeptide durch Umfällen mit Soda oder Pottasche erhalten werden.

Für die eigentliche Schotten-Baumann-Acylierung legt man üblicherweise die Peptidlösung vor, stellt alkalisch ein und läßt dann bei einer Temperatur von 40 bis 60°C eine solche Menge Fettsäurechlorid zulaufen, daß im Endprodukt ein Gesamtstickstoffgehalt im Bereich von 1,8 bis 4,1 und insbesondere 2,0 bis 3,5 erreicht wird. Die Berechnung der erforderlichen Menge hängt vom durchschnittlichen Molekulargewicht des Proteinhydrolysats ab und kann vom Fachmann leicht errechnet werden, ohne daß dieser hierzu erfinderisch tätig werden muß. Die bei der Kondensation freigesetzte Salzsäure wird durch Zugabe von Alkali abgefangen. Nachdem die Zugabe von Fettsäurechlorid abgeschlossen ist, läßt man 1 bis 2 h nachreagieren und kann dann das Proteinfettsäurekondensat auf den gewünschten Feststoffgehalt und pH-Wert einstellen.

### Fettsäurebasis

Die als Emulgatoren im Sinne der Erfindung in Betracht kommenden Kondensate stellen formal Acylierungsprodukte von Proteinhydrolysaten mit aliphatischen Fettsäuren der Formel (I) dar,

**R**^{**1**}**CO-OH** (I)

in der R¹CO für einen aliphatischen Acylrest mit 6 bis 22, vorzugsweise 12 bis 18 Kohlenstoffatomen steht. Wie schon beschrieben, wird der Fettacylrest jedoch nicht über die Fettsäuren, sondern die Fettsäurechloride in die Kondensate eingeführt. Wenn also im folgenden ausgeführt wird, von welchen Fettsäuren sich die Proteinfettsäurekondensate ableiten können, dann ist damit die Lehre zum technischen Handeln verknüpft, zu ihrer Herstellung die entsprechenden Fettsäurechloride einzusetzen.

Beispiele für Fettsäuren, von denen sich die Proteinfettsäurekondensate formal ableiten können, sind: Capronsäure, Caprylsäure, 2-Ethylhexansäure, Isononansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die beispielsweise durch Druckspaltung von Fetten und Ölen oder Reduktion von Aldehyden aus der Roelen'schen Oxosynthese erhältlich sind.

Die Proteinfettsäurekondensate können in Form ihrer Alkali-, Erdalkali- und/oder Ammoniumsalze, vorzugsweise als Natrium-, Magnesium- und/oder Calciumsalze eingesetzt werden.

### Kosmetische und pharmazeutische Zubereitungen

Die erfindungsgemäßen kosmetischen und pharmazeutischen Zubereitungen können die hochacylierten Proteinfettsäurekondensate in Mengen von 0,5 bis 5, vorzugsweise 1 bis 4 Gew.-% - bezogen auf die Zubereitungen - enthalten.

In einer bevorzugten Ausführungsform der Erfindung werden den hochacylierten Proteinfettsäurekondensaten Polyole, beispielsweise Ethylenglycol, Diethylenglycol, Triethylenglycol, Propylenglycol, Dipropylenglycol, technische Oligoglyceringemische mit einem durchschnittlichen Eigenkondensationsgrad von 1,5 bis 10, Trimethylolpropan, Pentaerythrit, Dipentaerythrit, Alkyloligoglucoside auf Basis von Fettalkoholen mit 8 bis 18 Kohlenstoffatomen, Zuckeralkohole wie Mannit oder Sorbit, Kohlenhydrate wie beispielsweise Glucose oder Aminozucker wie beispielsweise Glucamin oder N-Methylglucamin. Vorzugsweise wird als Polyol Glycerin eingesetzt. Die erfindungsgemäßen Zubereitungen können die als Coemulgatoren in Betracht kommenden Polyole in Mengen von 2 bis 15, vorzugsweise 5 bis 10 Gew.-% - bezogen auf die Zubereitungen - enthalten.

Die kosmetischen und/oder pharmazeutischen Zubereitungen können als weitere Inhaltsstoffe beispielsweise Emulgatoren, Ölkomponenten, Fette und Wachse, Verdickungsmittel, biogene Wirkstoffe, Filmbildner, Duftstoffe, Farbstoffe, Perlglanzmittel, Konservierungsmittel und pH-Regulatoren enthalten.

Als Überfettungsmittel können Substanzen wie beispielsweise polyethoxylierte Lanolinderivate, Lecithinderivate und Fettsäurealkanolamide verwendet werden, wobei die letzteren gleichzeitig als Schaumstabilisatoren dienen. Geeignete **Verdickungsmittel** sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon sowie als **Konsistenzgeber** Fettsäuren, Fettalkohole und Monoglyceride. Unter biogenen Wirkstoffen sind beispielsweise Pflanzenextrakte, Eiweißhydrolysate und Vitaminkomplexe zu verstehen. Gebräuchliche Filmbildner sind beispielsweise Polyvinylpyrrolidon, Vinylpyrrolidon-Vinylacetat-Copolymerisate, Polymere der Acrylsäurereihe, quaternäre Cellulose-Derivate und ähnliche Verbindungen. Als **Konservierungsmittel** eignen sich z.B. Formaldehydlösung, p-Hydroxybenzoat oder Sorbinsäure. Als **Perlglanzmittel** kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen** For**schungsgemeinschaft, veröffentlicht im Verlag Chemie, Weinheim, 1984,** zusammengestellt sind. Diese Farbstoffe werden üblicherweise in Konzentrationen von 0,001 bis 0,1 Gew.-%, bezogen auf die gesamte Mischung, eingesetzt.

### Gewerbliche Anwendbarkeit

Hochacylierte Proteinfettsäurekondensate, die einen Gesamtsrickstoffgehalt im Bereich von 2,0 bis 3,5 Gew.-% aufweisen, besitzen ausgezeichnete Emulgiereigenschaften. Bei ihrer Verwendung lassen sich lagerstabile Emulsionen einer konstanten Viskosität herstellen. Niedrigacylierte Proteinfettsäurekondensate nach dem Stand der Technik, die einen Gesamtstickstoffgehalt von > 4 Gew.-% besitzen, sind als Emulgatoren nachweislich ungeeignet.

Ein weiterer Gegenstand der Erfindung betrifft daher die Verwendung der genannten hochacylierten Proteinfettsäurekondensate als Emulgatoren für die Herstellung von kosmetischen und/oder pharmazeutischen Zubereitungen.

Die folgenden Beispiele sollen den Gegenstand der Erfindung näher erläutern, ohne ihn darauf einzuschränken.

### Beispiele

### I. Eingesetzte Proteinfettsäurekondensate

A1) Kaliumsalz eines Acylierungsproduktes von tierischem Kollagen (durchschnittliches Molekulargewicht 2500) mit C_{12/14}-Kokosfettsäure.
A2) Kaliumsalz eines Acylierungsproduktes von Sojaprotein (durchschnittliches Molekulargewicht 3000) mit C_{12/14}-Kokosfettsäure.
B1) Kaliumsalz eines Acylierungsproduktes von tierischem Kollagen (durchschnittliches Molekulargewicht 3000) mit C_{12/14}-Kokosfettsäure.

Die Kenndaten der drei Produkte sind in Tab.1 zusammengefaßt:

**Tab.1**

| Kenndaten der Produkte | | | |
|---|---|---|---|
| **Produkt** | **Lipidextrakt** **Gew.-%** | **Fettsäure im** **Lipidextrakt** **Gew.-%** | **Gesamtstickstoff** **Gew.-%** |
| A1 | 19,1 | 53,8 | 2,6 |
| A2 | 17,1 | 50,0 | 3,5 |
| B1 | 18,6 | 19,1 | 5,0 |

Die Produkte A1 und A2 sind erfindungsgemäß, das Produkt B1 dient dem Vergleich.

### II. Stabilitätsuntersuchungen

Die folgenden Emulsionen wurden nach einer Lagerzeit von 1 bis 12 Wochen bei 20°C hinsichtlich ihrer Viskosität und Stabilität beurteilt. Die Ergebnisse sind in Tab.2 zusammengefaßt:

**Tab.2**

| Lagerstabilität; Prozentangaben als Gew.-% | | | |
|---|---|---|---|
| **Komponenten** | **Beispiele** | | |
| | 1 | 2 | V1 |
| A1 | 3,5 % | - | - |
| A2 | - | 3,5 % | - |
| B1 | - | - | 3,5 % |
| Cetiol^{(R)} LC | 10,0 % | 10,0 % | 10,0 % |
| Cetiol^{(R)} OE | 5,0 % | 5,0 % | 5,0 % |
| Cutina^{(R)} GMS | 7,0 % | 7,0 % | 7,0 % |
| Lanette^{(R)} O | 2,0 % | 2,0 % | 2,0 % |
| Glycerin (86 Gew.-%ig) | 3,0 % | 3,0 % | 3,0 % |
| Wasser | 69,5 % | 69,5 % | 69,5 % |
| Viskosität*( 1 Woche) | 4000 | 4000 | 7200 |
| Viskosität*( 8 Wochen) | 4400 | 4100 | 9600 |
| Stabilität (12 Wochen) | stabil | stabil | nicht stabil |

| | | | |
|---|---|---|---|
| *) Viskosität nach Brookfield, Spindel 5, 10 Upm, [mPas] | | | |

### III. Beispielrezepturen (Wasser ad 100 Gew.-%)

### Beispiel 3: Tagespflegelotion

| | |
|---|---|
| A1 | 2,0 Gew.-% |
| Cetiol^{(R)} 868 | 7,0 Gew.-% |
| Cutina^{(R)} GMS | 5,0 Gew.-% |
| Cutina^{(R)} CP | 1,0 Gew.-& |
| Lanette^{(R)} O | 1,0 Gew.-% |
| Novata^{(R)} AB | 1,0 Gew.-% |
| Myritol^{/R)} GTEH | 7,0 Gew.-% |
| Glycerin (86 Gew.-%ig) | 5,0 Gew.-% |
| Carbopol^{(R)} 981 (2 Gew.-%ig) | 10,0 Gew.-% |

### Beispiel 4: Sonnenschutzlotion

| | |
|---|---|
| A2 | 1,5 Gew.-% |
| Cetiol^{(R)} S | 8,0 Gew.-% |
| Eutanol^{(R)}G | 2,0 Gew.-% |
| Lanette^{(R)} O | 1,0 Gew.-% |
| Novata^{(R)} AB | 1,0 Gew.-% |
| Vitamin E | 3,0 Gew.-% |
| Sheabutter | 1,0 Gew.-% |
| Titandioxid | 2,0 Gew.-% |
| Cyclomethicone | 6,0 Gew.-% |
| Neo Heliopan^{(R)} BB | 1,5 Gew.-% |
| Neo Heliopan^{(R)} E 1000 | 1,0 Gew.-% |
| Neo Heliopan^{(R)} Hydro | 1,0 Gew.-% |
| Carbopol^{(R)} 981 (2 Gew.-%ig) | 20,0 Gew.-% |
| Ethanol | 5,0 Gew.-% |

### IV. Eingesetzte Inhaltsstoffe

**Tab.3**

| Eingesetzte Inhaltsstoffe(*) | | | |
|---|---|---|---|
| **Bezeichnung** | | **Herst.** | **CTFA-Registrierung** |
| Carbopol | 981 | Goodrich | Polyacrylate |
| Cetiol | LC | Henkel | Coco-Caprylate/Caprate |
| | OE | | Dicaprylether |
| | S | | Dioctylcyclohexane |
| | 868 | | Octylstearate |
| Cutina | CP | Henkel | Cetyl palmitate Glyceryl stearate |
| | GMS | | |
| Eutanol | G | Henkel | Octyldodecanol |
| Lanette | O | Henkel | Cetearylalkohol |
| Neo Heliopan | B | Haarmann & Reimer | Lichtschutzmittel |
| | E 1000 | | |
| | Hydro | | |
| Novata | AB | Henkel | Coco glycerides |
| Myritol | GTEH | Henkel | Glyceryl trioctanoate |

| | | | |
|---|---|---|---|
| (*) Bei den angegebenen Bezeichnungen handelt es sich um eingetragene Warenzeichen. | | | |

## Patentansprüche

1. Kosmetische und/oder pharmazeutische Zubereitungen, **dadurch gekennzeichnet, daß** sie als Emulgatoren hochacylierte Proteinfettsäurekondensate enthalten, die einen Gesamtstickstoffgehalt - bezogen auf die Acylierungsprodukte - im Bereich von 2,0 bis 3,5 Gew.% aufweisen, wobei man Acylierungsprodukte auf Basis von Sojaprotein-, Mandelprotein-, Weizenprotein- und/oder Kartoffelproteinhydrolysaten einsetzt.

2. Zubereitungen nach Anspruch 1, **dadurch gekennzeichnet, daß** man Acylierungsprodukte auf Basis von Proteinhydrolysaten einsetzt, die ein durchschnittliches Molekulargewicht im Bereich von 600 bis 4000 aufweisen.

3. Zubereitungen nach den Ansprüchen 1 und 2, **dadurch gekennzeichnet, daß** man Acylierungsprodukte von Proteinhydrolysaten mit Fettsäuren der Formel (I) einsetzt,
**R**^{**1**}**CO-OH (I)**
in der R¹CO für einen aliphatischen Acylrest mit 6 bis 22 Kohlenstoffatomen steht.

4. Zubereitungen nach den Ansprüchen 1 bis 3, **dadurch gekennzeichnet, daß** man die Acylierungsprodukte in Form ihrer Alkali, Erdalkali- und/oder Ammoniumsalze einsetzt.

5. Zubereitungen nach den Ansprüchen 1 bis 4, **dadurch gekennzeichnet, daß** se die Acylierungsprodukte in Mengen von 0,5 bis 5 Gew.-% - bezogen auf die Zubereitungen - enthalten.

6. Zubereitungen nach den Ansprüchen 1 bis 5, **dadurch gekennzeichnet, daß** sie als Co-Emulgatoren 2 bis 15 Gew.% - bezogen auf die Zubereitungen - Polyole enthalten.

7. Verwendung von hochacylierten Proteinfettsäurekondensaten, die einen Gesamtstickstoffgehalt - bezogen auf die Acylierungsprodukte - im Bereich von 1,8 bis 4,1 Gew.% aufweisen als Emulgatoren für die Herstellung von Cremes und Lotionen.

## Claims

1. Cosmetic and/or pharmaceutical preparations, **characterized in that** they contain as emulsifiers highly acylated protein fatty acid condensates with a total nitrogen content - based on the acylation products - of 2.0 to 3.5% by weight, acylation products based on soya protein, almond protein, wheat protein and/or potato protein hydrolyzates being used.

2. Preparations as claimed in claim 1, **characterized in that** acylation products based on protein hydrolyzates with an average molecular weight of 600 to 4,000 are used.

3. Preparations as claimed in claims 1 and 2, **characterized in that** acylation products of protein hydrolyzates with fatty acids corresponding to formula (I):
R¹CO-OH (I)
in which R¹CO is an aliphatic C₆₋₂₂ acyl group, are used.

4. Preparations as claimed in claims 1 to 3, **characterized in that** the acylation products are used in the form of their alkali metal, alkaline earth metal and/or ammonium salts.

5. Preparations as claimed in claims 1 to 4, **characterized in that** they contain the acylation products in quantities of 0.5 to 5% by weight, based on the preparations.

6. Preparations as claimed in claims 1 to 5, **characterized in that** they contain 2 to 15% by weight, based on the preparations, of polyols as co-emulsifiers.

7. The use of the highly acylated protein fatty acid condensates with a total nitrogen content - based on the acylation products - of 1.8 to 4.1 % by weight as emulsifiers for the production of creams and lotions.

## Revendications

1. Préparations cométiques et/ou pharmaceutiques,
**caractérisées en ce qu'**
elles contiennent comme émulsifiants des condensats d'acides gras protéiques hautement acylés, qui présentent une teneur totale en azote - par rapport aux produits d'acylation - comprise entre 2,0 et 3,5 % en poids,
en utilisant des produits d'acylation à base d'hydrolysats de protéine de soja, de protéine d'amande, de protéine de blé, et/ou de protéine de pomme de terre.

2. Préparations selon la revendication 1,
**caractérisées en ce qu'**
on utilise des produits d'acylation à base d'hydrolysats de protéines qui présentent un poids moléculaire moyen compris entre 600 et 4000.

3. Préparations selon les revendications 1 ou 2,
**caractérisées en ce qu'**
on utilise des produits d'acylation d'hydrolysats de protéines avec des acides gras de formule (I),
R¹CO-OH (I)
dans laquelle R¹CO représente un radical acyle aliphatique ayant 6 à 22 atomes de carbone.

4. Préparations selon les revendications 1 à 3,
**caractérisées en ce qu'**
on utilise les produits d'acylation sous la forme de leurs sels de métaux alcalins, de métaux alcalino-terreux et/ou d'ammonium.

5. Préparations selon les revendications 1 à 4,
**caractérisées en ce qu'**
elles contiennent les produits d'acylation en des quantités de 0,5 à 5 % en poids - par rapport aux préparations.

6. Préparations selon les revendications 1 à 5,
**caractérisées en ce qu'**
elles contiennent comme co-émulsifiants de 2 à 15 % en poids de polyols par rapport aux préparations.

7. Utilisation de condensats d'acides gras protéiques hautement acylés dont la teneur globale en azote rapportée aux produits d'acylation est de 1,8 à 4,1 % en poids, comme émulsifiants pour la fabrication de crèmes et lotions.
